# EUROPEAN PATENT APPLICATION

(11) **EP 4 400 113 A1**
(43) Date of publication of application: **17.07.2024**
(21) Application number: 21953423.7
(22) Date of filing: 27.12.2021
(51) Int. Cl.: A61K 38/00, A61K 48/00, A61K 45/00, C12N 15/867, C12N 5/10

(54) **HYPOXIA-TRIGGERED ARTIFICIAL TRANSCRIPTION FACTOR AND HYPOXIA-TRIGGERED TRANSCRIPTION CONTROL SYSTEM, AND APPLICATION THEREOF**

(30) Priority: 13.08.2021 CN 202110930268
(71) Applicant: Shanghai Sinobay Biotechnology Co., Ltd., Shanghai 201506 (CN)
(72) Inventor: XU, Jianqing, Shanghai 201506 (CN); ZHANG, Xiaoyan, Shanghai 201506 (CN); LIAO, Qibin, Shanghai 201506 (CN); DING, Xiangqing, Shanghai 201506 (CN)
(74) Representative: Uexküll & Stolberg
(86) International application number: PCT/CN2021/141636
(87) International publication number: WO 2023/015822

(57) **Abstract**

The present invention provides a hypoxia-triggered artificial transcription factor (HATF), and further provides a hypoxia-triggered transcription control system. The transcription control system comprises a nucleic acid sequence encoding the HATF, and a recognition element (RE). The hypoxia-triggered transcription control system comprises two sets of transcription control units linked upstream and downstream, wherein the upstream transcription control unit comprises a hypoxia-triggered transcription reaction element for controlling the HATF and a nucleic acid sequence encoding the HATF, and the downstream transcription control unit comprises an RE and a gene of interest. Co-regulation by the artificial transcription factor HATF and the recognition element RE can increase the expression of the gene of interest by a factor of one hundred.

## Description

### Technical Field

The present invention belongs to the field of biomedical technology, and particularly relates to a hypoxia-triggered artificial transcription factor. The present invention further relates to a hypoxia-triggered transcription control system including the hypoxia-triggered artificial transcription factor, and use thereof in the treatment of hypoxic tumors.

### Background Art

Immune cell therapies for tumors, such as T cell receptor T cells (TCR-T cells) and chimeric antigen receptor T cells (CAR-T cells), have shown good anti-tumor effects in clinical studies. However, in clinical trials and applications, new questions continue to arise with cell therapies. In addition to the difficulty of entering dense solid tumors, common adverse reactions include off-target effects due to tumor-specific antigen deficiency, potentially fatal neurotoxicity, allergic reactions and other side effects, which limit the clinical applications of cell therapies for solid tumors. Therefore, it is necessary to further develop an immune cell therapy that can be regulated and precisely targeted at tumors, in order to reduce the damages to normal tissues.

It has been found that hypoxia is a common feature of many solid tumors due to insufficient blood supply caused by abnormal vascular structures in solid tumors and excessive proliferation of tumor cells, creating a relatively hypoxic tumor microenvironment, with an oxygen level in tumor tissues being often less than 2%. Aiming at the hypoxic microenvironment in solid tumors, the development of tumor killing technologies that activate TCR-T cells, CAR-T cells or genetically engineered T cells through hypoxia signals is expected to improve the specificity of tumor therapy.

Previous studies have shown that fusing an oxygen-dependent degradation domain (ODD) of hypoxia-inducible factors 1-alpha (HIF-1α) into a chimeric antigen receptor can degrade the chimeric antigen receptor in a normoxic environment and enrich the chimeric antigen receptor in a hypoxic environment, thereby recognizing and killing tumor cells. However, an induction level of the chimeric antigen receptor fused with the oxygen-dependent degradation domain in the hypoxic environment is still at a low level, which is not conducive to the control of tumors with large antigenic heterogeneity, especially solid tumors with low tumor antigen expression. In addition, the current ODD-regulated hypoxia-sensitive CAR-T cells still have a high level of background leakage and can produce a certain level of non-hypoxia-dependent killing, resulting in safety concerns. In addition, although CAR-T cells driven by hypoxia-triggered promoters have a certain ability to respond to oxygen concentration, they also have a higher level of background leakage, thereby resulting in a higher level of non-hypoxia-dependent killing, which may produce on-target off-tumor toxicity caused by targeting non-tumor cells.

Therefore, it is necessary to further develop more sensitive and efficient hypoxia-triggered artificial transcription factors, so as to develop safer and more effective hypoxia-sensitive treatment methods, which can not only improve the safety of cell therapy, but also effectively control tumor growth.

### Summary of the Invention

An object of the present invention is to provide a hypoxia-triggered transcription control system against the defects of the prior art. The hypoxia-triggered transcription control system provided by the present invention includes two sets of transcription control units linked upstream and downstream. The present invention further provides use of the hypoxia-triggered transcription control system. The transcription control system provided by the present invention is composed of two sets of linked transcription control units, forming upstream and downstream two-stage amplifications for achieving high sensitivity to hypoxia. In addition, the system can realize the preparation of a medicament for tumor treatment and precision treatment by carrying different genes of interest. Compared with the prior art, the hypoxia-triggered transcription control system of the present invention is efficiently and inducibly expressed under hypoxia, thereby improving the strictness and sensitivity of hypoxia triggering. In addition, with the help of the transcription control system of the present invention, the higher expression of genes of interest can be driven by using a small amount of transcription factors, thereby further amplifying its sensitivity to hypoxia.

The objects of the present invention are achieved by the following technical solutions.

In an aspect, the present invention provides a hypoxia-triggered artificial transcription factor (HATF), the HATF comprising:
(1) a sequence shown in SEQ ID NO: 1;
(2) a sequence having one or more amino acid substitutions, deletions or additions compared with the sequence shown in SEQ ID NO: 1; or
(3) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in SEQ ID NO: 1.

In another aspect, the present invention further provides a nucleic acid sequence encoding the HATF.

In yet another aspect, the present invention provides a recognition element (RE) of the HATF, the RE comprising a core sequence selected from:
(1) a sequence shown in SEQ ID NO: 2, or its complementary sequence;
(2) a sequence hybridized to the sequence shown in SEQ ID NO: 2 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 2; or
(4) a sequence obtained by derivatization of the sequence shown in SEQ ID NO: 2 by deletion, substitution, insertion, or addition of one or more nucleotides.

Preferably, the RE comprises a plurality of copies of the core sequence and a minimal promoter; more preferably, the plurality of copies are two copies, three copies, four copies, five copies, six copies, seven copies, eight copies, nine copies, or ten copies; and further preferably, the recognition element (RE) comprises five or six copies of the core sequence and a minimal promoter.

Still further preferably, the RE comprises a sequence selected from:
(1) a sequence shown in SEQ ID NO: 3, or its complementary sequence;
(2) a sequence hybridized to the sequence shown in SEQ ID NO: 3 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 3; or
(4) a sequence obtained by derivatization of the sequence shown in SEQ ID NO: 3 by deletion, substitution, insertion, or addition of one or more nucleotides.

Most preferably, the RE is shown in SEQ ID NO: 3.

The HATF provided by the present invention is a novel artificial transcription factor. The inventors of the present invention have found that co-regulation by this HATF and its matching RE can increase the expression of a gene of interest by a factor of more than one hundred, which is far greater than the upregulation achieved by the conventional artificial transcription factor and its reaction element.

In another aspect, the present invention provides a hypoxia-triggered transcription control system. The transcription control system comprises a nucleic acid sequence encoding a HATF, and a RE.

The hypoxia-triggered transcription control system according to the present invention comprises two sets of transcription control units linked upstream and downstream, wherein the upstream transcription control unit comprises a hypoxia-triggered transcription reaction element (HRTE) for controlling the HATF, and a nucleic acid sequence encoding the HATF; and the downstream transcription control unit comprises an RE and a gene of interest (GOI).

The HATF is triggered by hypoxia during both RNA transcription and protein translation, that is, the hypoxia-triggered artificial transcription factor is not expressed or expressed at a low level under normoxia, but is efficiently and inducibly expressed under hypoxia, thereby improving the strictness and sensitivity of hypoxia triggering.

In the hypoxia-triggered transcription control system according to the present invention, the transcription control units linked upstream and downstream are nonlinearly connected in series, and located at two vectors; and preferably, the transcription control system has a combined form as shown in any one of the following formulas:
HATF-HRTE and RE-GOI;
HATF-HRTE and GOI-RE;
HRTE-HATF and GOI-RE; or
HRTE-HATF and RE-GOI.

Alternatively, the transcription control units linked upstream and downstream are linearly connected in series, and located at the same vector; and preferably, the transcription control system has a combined form as shown in any one of the following formulas:
HATF-HRTE-RE-GOI;
HATF - HRTE-GOI - RE;
HRTE-HRTE-GOI;
HRTE- HATF -GOI - RE;
RE-GOI-HATF-HRTE;
RE-GOI - HRTE- HATF;
GOI-RE-HATF-HRTE; or
GOI-RE-HRTE-HATF.

In the hypoxia-triggered transcription control system according to the present invention, the HRTE is selected from a flanking region of a hypoxia-inducible gene, such as a vascular endothelial growth factor gene, an erythropoietin gene or a glycolytic enzyme gene, and a core sequence of the HRTE is 5'-(A/G)CGT(G/C)-3'.

In the hypoxia-triggered transcription control system according to the present invention, the HRTE comprises a core sequence selected from:
(1) a sequence shown in SEQ ID NO: 4, or its complementary sequence;
(2) a sequence hybridized to the sequence shown in SEQ ID NO: 4 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 4; or
(4) a sequence obtained by derivatization of the sequence shown in SEQ ID NO: 4 by deletion, substitution, insertion, or addition of one or more nucleotides.

Preferably, the HRTE includes a plurality of copies of the core sequence and a minimal promoter; more preferably, the plurality of copies are two copies, three copies, four copies, five copies, six copies, seven copies, eight copies, nine copies or ten copies; and further preferably, the HRTE comprises five or six copies of the core sequence and a minimal promoter.

Still further preferably, the HRTE comprises a sequence selected from:
(1) a sequence shown in SEQ ID NO: 5, or its complementary sequence;
(2) a sequence hybridized to the sequence shown in SEQ ID NO: 5 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 5; or
(4) a sequence obtained by derivatization of the sequence shown in SEQ ID NO: 5 by deletion, substitution, insertion, or addition of one or more nucleotides.

Most preferably, the HRTE is shown in SEQ ID NO: 5.

In a specific embodiment, the artificial transcription factor HATF according to the present invention has an amino acid sequence shown in SEQ ID NO: 1, and the recognition element RE of the HATF has a key nucleic acid sequence shown in SEQ ID NO: 3. Co-regulation by the HATF and the RE can increase the expression of a gene of interest (red fluorescent protein mCherry) by a factor of one hundred, which is much superior to the 10-fold up-regulation achieved by the conventional artificial transcription factor (GAL4) and its reaction element (UAS).

In the hypoxia-triggered transcription control system according to the present invention, the gene of interest GOI is a functional gene; and
preferably, the GOI is one or more selected from:
a cytokine and a chemokine, such as GM-CSF, lFN-α/β/γ, IL-2, IL-3, IL-7, IL-12, IL-15, IL-21, IL-33, IL-35, IL-37, CCL4, CCL20, CXCL9, CXCL10, CXCL11, CXCL12, CXCL13, MIP-1α, MIP-1β, and the like;
a cytotoxic molecule, such as TNF-α, bispecific T cell engagers (BiTE) against different antigen targets, chimeric antigen receptors (CAR) against different antigen targets, an apoptotic gene, a pyroptosis gene, a toxin, and the like;
agonistic antibodies against different antigen targets, such as an anti-CD28 antibody, an anti-4-1BB antibody, an anti-ICOS antibody, an anti-GITR antibody, an anti-OX40 antibody and an anti-CD27 antibody; or blocking antibodies against different antigen targets, such as a CTLA-4 antibody, a PD-1 antibody, a PD-L1 antibody, a LAG-3 antibody, a Tim3 antibody, and the like; or a combination of any two or more of the above.

Preferably, the corresponding antigen targets are broad-spectrum universal targets expressed on different cell membranes, including but not limited to: AXL, EGFR, MHC, CD24, CD47, FAP, CD147, HER-2, CD55, CD59, ROR1, ROR2, CD133, CD44v6, CD44v7, CD44v8, CD126, CD171, CEA, EpCAM, TAG72, IL-13Rα, EGFRvIII, GD2, GD3, FRα, PSCA, PSMA, GPC3, CAIX, Claudin18.2, VEGFR2, PD-L1, PD-L2, MSLN, MUC1, c-Met, FOLR1, B7-H3, Trop2, and the like; and
preferably, an expression cassette of the GOI comprises an amino acid sequence shown in any one of SEQ ID NOs: 6-8, including a red fluorescent protein mCherry shown in SEQ ID NO: 6, a chimeric antigen receptor (CAR) binding to a tumor antigen HER2 shown in SEQ ID NO: 7, and a bispecific T-cell engager (BiTE) of CD47/CD3 shown in SEQ ID NO: 8.

The present invention further provides a nucleic acid sequence, comprising the hypoxia-triggered transcription control system of the present invention; and
preferably, the nucleic acid sequence is shown in SEQ ID NO: 9.

The present invention further provides a vector, comprising the nucleic acid sequence; preferably, the vector is selected from a plasmid, a retroviral vector, a lentiviral vector, an adenoviral vector, an adeno-associated viral vector, a vaccinia virus vector, a herpes simplex virus vector, a forest encephalitis virus vector, a poliovirus vector, a Newcastle disease virus vector, a transposon or a combination of one or more thereof; and more preferably, the vector is the lentiviral vector.

The present invention further provides a host cell. The host cell comprises the vector or the nucleic acid molecule is integrated into a chromosome of the host cell.

Preferably, the host cell is an isolated human-derived cell, including an embryonic stem cell, a umbilical cord blood-derived stem cell, an induced pluripotent stem cell, a hematopoietic stem cell, a mesenchymal stem cell, an adipose-derived stem cell, a T cell, an NK cell, an NKT cell or a macrophage; more preferably, the human-derived cell is a genetically engineered immune cell; further preferably, the genetically engineered immune cell is a T cell, an NK cell, an NKT cell or a macrophage; and still further preferably, the genetically engineered immune cell is a T cell.

Most preferably, the genetically engineered immune cell is selected from the group consisting of:
a chimeric antigen receptor T cell (CAR-T cell);
a chimeric antigen receptor NK cell (CAR-NK cell);
a chimeric antigen receptor NKT cell (CAR-NKT cell);
a chimeric antigen receptor macrophage (CAR- mø); and
a T cell receptor T cell (TCR-T cell).

In a preferred embodiment, the genetically engineered immune cell is a CAR-T cell. The hypoxia-triggered transcription control system of the present invention is introduced into the CAR-T cell by transfection or transduction via the vector, thereby inducing the expression of a red fluorescent protein mCherry, a chimeric antigen receptor or a bispecific T cell engager in a hypoxic environment.

The present invention further provides a method for preparing a genetically engineered cell comprising the hypoxia-triggered transcription control system, wherein the method comprises the steps of: introducing a vector comprising the hypoxia-triggered transcription control system of the present invention into a host cell, thereby obtaining the genetically engineered cell.

In a preferred embodiment, the introduction may be performed simultaneously, in a chronological order, or in sequence.

The present invention further provides use of the hypoxia-triggered artificial transcription factor HATF, the recognition element RE of the HATF, the hypoxia-triggered transcription control system, the nucleic acid molecule, the vector and the host cell in the preparation of a medicament or a formulation for treating hypoxic diseases, ischemic diseases, or cancers.

Preferably, the cancer is a solid tumor; and more preferably, the solid tumor is one or more selected from neuroblastoma, lung cancer, breast cancer, esophageal cancer, gastric cancer, liver cancer, cervical cancer, ovarian cancer, kidney cancer, pancreatic cancer, nasopharyngeal cancer, small bowel cancer, large bowel cancer, colorectal cancer, bladder cancer, bone cancer, prostate cancer, thyroid cancer or brain cancer.

A method for treating hypoxic diseases, ischemic diseases or cancers comprises: administrating to a subject in need thereof a therapeutically effective amount of the hypoxia-triggered artificial transcription factor HATF, the recognition element RE of the HATF, the hypoxia-triggered transcription control system, the nucleic acid molecule, the vector, the host cell, a combination of the vector and the cell, or a combination of the above with other therapeutic drugs and technologies, including but not limited to a PD-1 antibody, a PD-L1 antibody, a CTLA-4 antibody, a TIGIT antibody, a Tim-3 antibody, and a LAG-3 antibody.

The present invention provides a hypoxia-triggered artificial control factor and a transcription control system comprising the same. The present invention further provides use of the hypoxia-triggered artificial control factor in the treatment of hypoxic diseases such as solid tumors, especially the use in CAR-T cell therapy of solid tumors.

Compared with the prior art, the present invention has the following advantages.
1. Co-regulation by the artificial transcription factor HATF of the present invention and the recognition element RE can increase the expression of the gene of interest by a factor of one hundred, which is much superior to the 10-fold up-regulation achieved by the conventional artificial transcription factor (GAL4) and its reaction element (UAS).
2. The artificial transcription factor HATF of the present invention is regulated by oxygen during both RNA transcription and protein translation. It is efficiently degraded in a normoxic environment, and is efficiently and inducibly expressed in a hypoxic environment, thereby ensuring the strictness and sensitivity of the hypoxia triggering of the system.
3. The artificial transcription factor HATF of the present invention can specifically bind to its matching recognition element RE to initiate the efficient transcription and expression of the gene of interest, so as to realize targeted delivery in the hypoxic environment and avoid serious toxic and side effects caused by systematic administration.
4. A protein encoded by the gene of interest in the hypoxia-triggered transcription control system of the present invention does not make any modification on a hypoxia-triggered functional domain, which can ensure that the function of the protein encoded by the gene of interest exerts its maximum activity without any interference.

It should be understood that the above technical features of the present invention and the technical features specifically described below (e.g., in examples) may be combined with each other within the scope of the present invention, thereby constituting a new or preferred technical solution. Due to limited space, these technical features are not repeated one by one.

### Brief Description of the Drawings

The embodiments of the present invention will be described in detail below with reference to the accompanying drawings. In drawings:

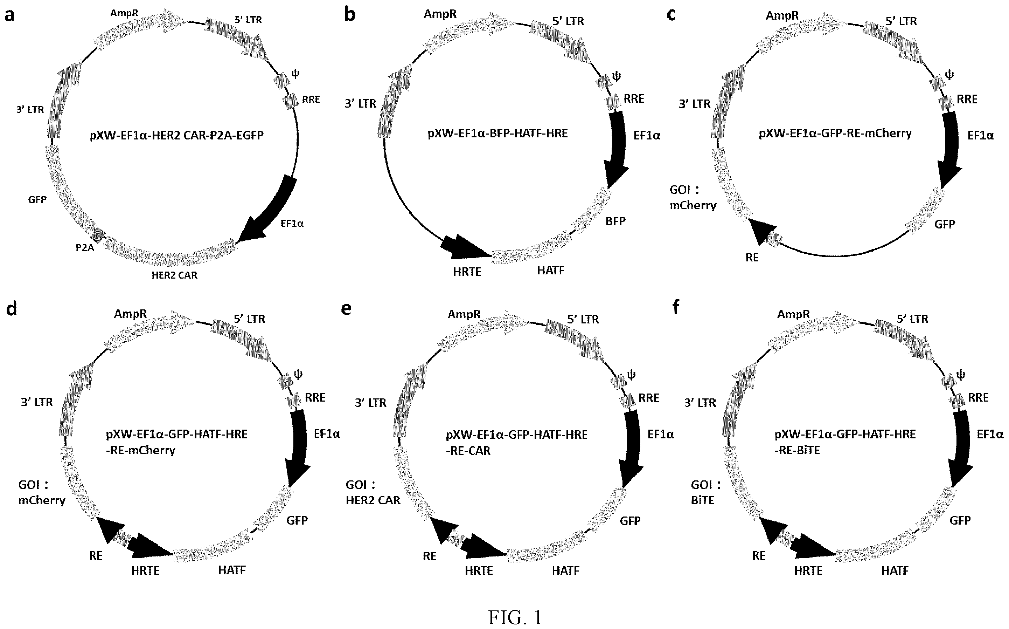

Fig. 1 shows the profiles of lentiviral expression plasmids carrying conventional HER2 CAR that is not triggered by hypoxia (Fig. 1a), a hypoxia-triggered transcription control system in a binary unit form (Figs. 1b-c), and a hypoxia-controlled transcription control system in a unary unit form (Figs. 1d-f), respectively. The hypoxia-triggered transcription control system in the binary unit form consists of HATF and HRTE, as well as RE and GOI provided by two lentiviral expression plasmids, while the hypoxia-triggered transcription control system in the unary unit form consists of HATF, HRTE, RE, and GOI provided by one lentiviral expression plasmid. The GOIs encode a red fluorescent protein mCherry (Fig. 1d), a chimeric antigen receptor (CAR) (Fig. 1e), or a bispecific T cell engager (BiTE) (Fig. 1f), respectively, where mCherry has an indicative function, and CAR and BiTE may be used for tumor immunotherapy.

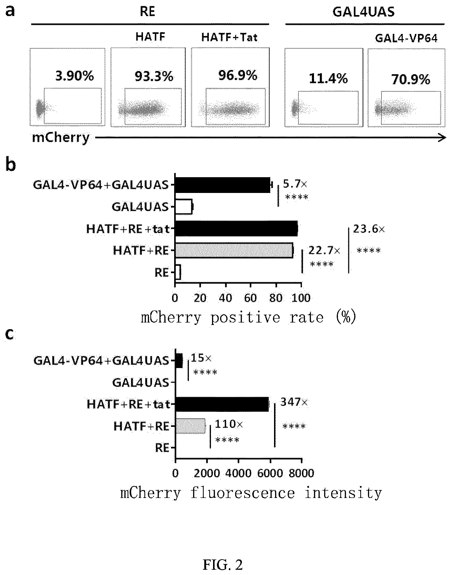

Fig. 2 shows that the expression of red fluorescent protein mCherry can be increased by a factor of more than one hundred by co-regulation of HATF and its matching RE, matchingwhich is much superior to the 10-fold up-regulation achieved by the conventional artificial transcription factor (GAL4-VP64) vector and its reaction element (GAL4 UAS). Fig. 2a shows a flow cytometry diagram of the expression of a gene of interest (mCherry) after 48 h of transfection. Fig. 2b and Fig. 2c show that an expression positive rate and fluorescence intensity of mCherry are significantly improved after 48 h of transfection, respectively, and the HATF of the present invention and the RE element matched with the HATF are significantly superior to the conventional artificial transcription factor and its matching reaction element in terms of background leakage and target induction level.

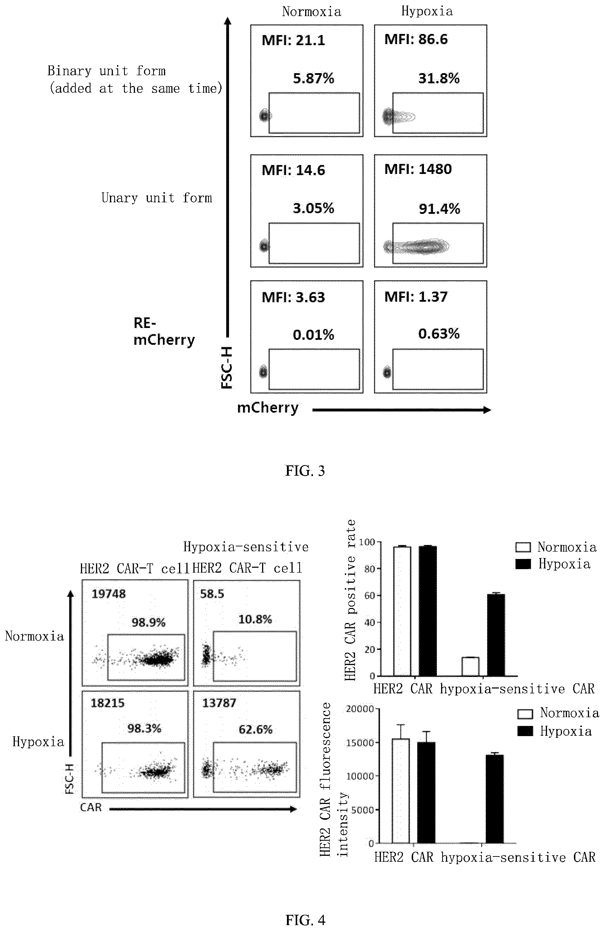

Fig. 3 shows that a lentiviral expression vector prepared for the lentiviral expression plasmid in Fig. 1 transduces a positive primary T cell, which can only detect a very low level of mCherry expression in a normoxic environment, while effectively inducing the mCherry expression under hypoxic conditions. In addition, the hypoxia induction level of the hypoxia-triggered transcription control system in the binary unit form introduced by dual virus vectors is much lower than that of the hypoxia-triggered transcription control system in the unary unit form introduced by a single virus vector.
Fig. 4 shows CAR-induced expression levels of engineered T cells (hypoxia-sensitive HER2 CAR-T cells) of the hypoxia-triggered transcription control system and conventional HER2 CAR-T cells at different oxygen concentrations. Flow cytometry results show that the hypoxia-sensitive HER2 CAR-T cells have good hypoxia induction characteristics, the expression amount of HER2 CAR is significantly increased under physiological hypoxia (1% O₂), and the expression level of hypoxia induction of HRTE2 CAR is comparable to the HER2 CAR expression level of the currently common HER2 CAR-T cells.

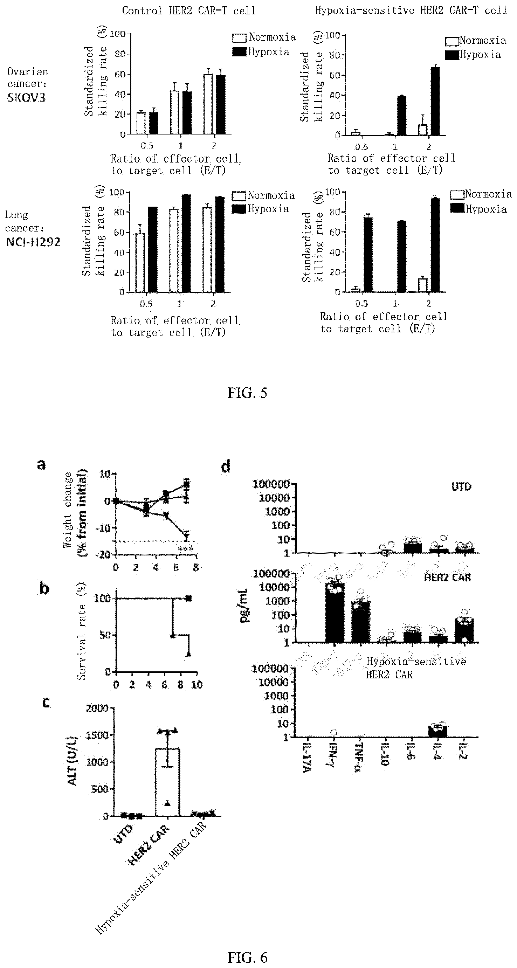

Fig. 5 shows that HER2 CAR-T cells (hypoxia-sensitive HER2 CAR-T cells) controlled by the hypoxia-triggered transcription control system selectively kill SKOV3 and NCI-H292 tumor cells expressed by HER2 antigens in a hypoxic environment, while the conventional HER2 CAR-T cells have no selective cell killing, and there is no difference between the normoxic environment and the hypoxic environment in terms of tumor cell killing efficiency.
Fig. 6 shows that the HER2 CAR-T cells (hypoxia-sensitive HER2 CAR-T cells) controlled by the hypoxia-triggered transcription control system are evaluated for their *in vivo* safety on a humanized mouse model of a human-derived HER2 target antigen. Only control HER2 CAR-T cells may cause a progressive decrease in body weight, increased mortality and impaired liver function of the mouse, and release a large number of cytokines related to T cell activation into the blood, while the hypoxia-sensitive HER2 CAR-T cells may not cause the above toxic and side effects.

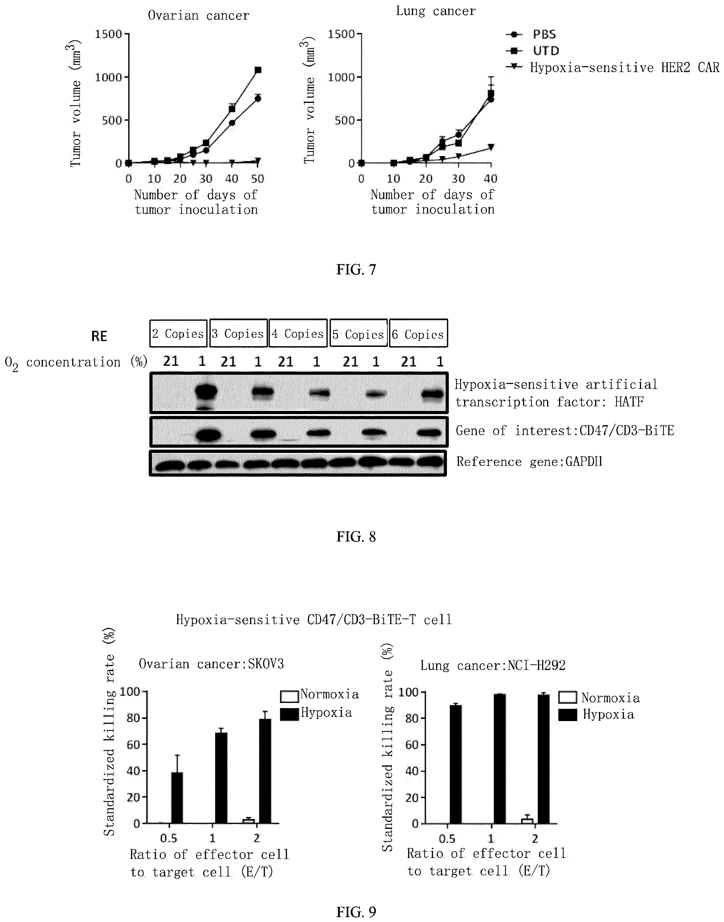

Fig. 7 shows that the HER2 CAR-T cells (hypoxia-sensitive HER2 CAR-T cells) controlled by the hypoxia-triggered transcription control system can effectively control the growth of ovarian cancer (SKOV3) and lung cancer (NCI-H292).
Fig. 8 shows that the hypoxia-triggered transcription control system regulates the expression of a bispecific T cell engager protein, that is, the hypoxia-sensitive artificial transcription factor is not expressed in a normoxic environment, and the artificial transcription factor inducibly expressed in the hypoxic environment may be combined with artificial transcription factor recognition elements with different copy numbers to drive the expression of a CD47/CD3 bispecific T cell engager protein.
Fig. 9 shows that BiTE-T cells (hypoxia-sensitive BiTE-T cells) controlled by the hypoxia-triggered transcription control system can selectively kill SKOV3 and NCI-H292 tumor cells in a hypoxic environment.

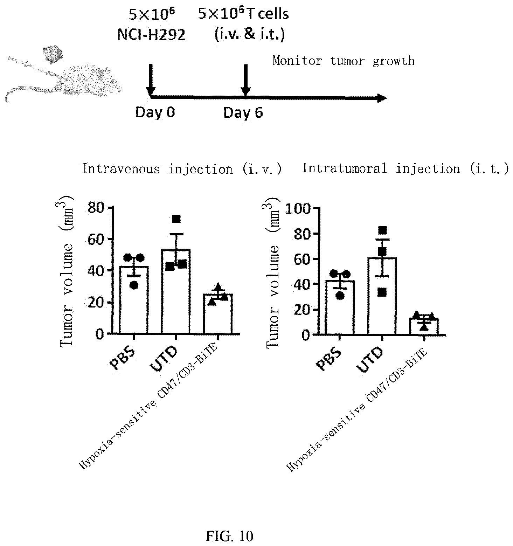

Fig. 10 shows that BiTE-T cells (hypoxia-sensitive BiTE-T cells) controlled by the hypoxia-triggered transcription control system by intravenous infusion and intratumoral injection can effectively inhibit tumor growth.

### Mode of Carrying Out the Invention

The following examples are used to illustrate the present invention, but not to limit the scope of the present invention.

Experimental methods used in the following examples were conventional experimental methods in the art unless otherwise specified. Experimental materials used in the following examples, unless otherwise specified, were purchased from conventional biochemical reagent sales companies, wherein:
DMEM medium and RPMI1640 medium were purchased from Corning, and lymphocyte medium X-VIVO 15 was purchased from Lonza.

A T cell growth medium, which was composed of a basal medium and cytokines, was prepared with reference to the Chinese invention patent CN201910163391.1. The basal medium was a lymphocyte medium X-VIVO 15, and the cytokines, i.e., 5 ng/mL IL-7, 10 ng/mL IL-15 and 30 ng/mL IL-21 were added. The cytokines IL-7 and IL-15 were purchased from R&D, and IL-21 was purchased from Nearshore Protein Technology Co., Ltd.

Fetal bovine serum was purchased from BI Inc.

A TurboFect Transfection Kit was purchased from Thermo Fisher Scientific.

A Lenti-X lentiviral concentrate reagent was purchased from Takara, Inc.

Gene synthesis was purchased from Shanghai Generay Bioengineering Co., Ltd.

A packaging plasmid psPAX2 and an envelope plasmid PMD2.G in blank lentiviral expression plasmids (pXW-EF1α-MCS-P2A-EGFP and pXW-EF1α-MCS) were purchased from Addgene, and pSV1.0-EGFP-ATR-RE, pSV1.0-EGFP-GAL4UAS, pSV1.0-HATF, pSV1.0-Tat and pSV1.0-GAL4-VP64 were purchased from Shanghai SINOBAY Biotechnology Co., Ltd.

Stable 3 chemically competent cells were purchased from Shanghai Weidi Biotechnology Co., Ltd.

An endotoxin-free plasmid miniprep kit and an endotoxin-free plasmid midiprep kit were purchased from OMEGA and Macherey Nagel, respectively.

A luciferase substrate was purchased from Promega Biotechnology Co., Ltd.

HEK293T cells, A549 lung cancer cells, SKOV3 ovarian cancer cells, and NCI-H292 lung cancer cells were purchased from ATCC in the United States. SKOV3-luc and NCI-H292-luc, which stably integrated firefly luciferase genes, were engineered by Shanghai SINOBAY Biotechnology Co., Ltd.

Severe combined immuno-deficient mice (B-NDG) were purchased from Biocytogen (Jiangsu) Gene Biotechnology Co., Ltd.

Replication-deficient adenovirus type 5 (HER2-Luc-Ad) loaded with a human-derived HER2 gene and firefly luciferase was purchased from GeneChem.

### Example 1: Construction of lentiviral expression plasmid

Nucleic acid sequences shown in SEQ ID NOs: 2-5 and 9 were synthesized by Shanghai Generay Bioengineering Co., Ltd. and cloned into blank lentiviral expression plasmids (pXW-EF1α-MCS-P2A-EGFP and pXW-EF1α-MCS) to obtain the following recombinant lentiviral expression plasmids:
pXW-EF1α-HER2 CAR-P2A-EGFP;
pXW-EF1α-BFP- HATF-HRTE;
pXW-EF1α-GFP-RE-mCherry;
pXW-EF1α-GFP-HATF-HRTE-RE-mCherry;
pXW-EF1α-GFP-HATF-HRTE-RE-CAR; and
pXW-EF1α-GFP-HATF-HRTE-RE-BiTE, and plasmid profiles were shown in Fig. 1.

### Example 2: Packaging, concentration and titer determination of lentivirus

### 2.1 Packaging of lentivirus

HEK293T cell treatment: 24 h before transfection, HEK293T cells in the logarithmic growth phase were collected, and inoculated in a 10 cm cell culture dish (6×10⁶ to 8×10⁶ cells); and the cells grew in 10 mL of complete DMEM medium, and cultured in a 37°C, 5% CO₂ cell incubator for 18-24 h, followed by plasma transfection till the cell density reached 70-90%.

HEK293T cell transfection: 1 mL of basal DMEM medium was added to a 15 mL centrifuge tube, and a transfected mixed solution was prepared according to a mass ratio of lentiviral expression plasmids to packaging plasmids to envelope plasmids of 1: 3: 1, with a total plasmid amount of 15 µg/dish. 30 µL of TurboFect transfection reagent was added according to a ratio of plasmids (µg) to a transfection reagent (µL) of 1: 2, incubated at room temperature for 15-20 min, then added to a dish plated with HEK293T cells, and continued to be cultured in a 37°C, 5% COz cell incubator for 48 h; a virus supernatant was then collected, and centrifuged at 1000×g, 4°C for 10 min; precipitates at the bottom of the tube were discarded; and a virus supernatant was collected.

### 2.2 Concentration of lentivirus

A 0.45 µm filter was used to further filter the virus supernatant collected by centrifugation, added with a Lenti-X lentiviral concentration reagent in 1/3 of the volume of the virus supernatant, inverted and mixed uniformly several times, incubated at 4°C overnight, and centrifuged at 2000×g, 4°C for 45 min, wherein a white precipitate was seen at the bottom of the centrifuge tube, which is concentrated virus particles. The supernatant was discarded carefully, and the white precipitate was resuspended with a blank RPMI1640 medium in 1/50-1/100 of the volume of the original virus supernatant, aliquoted and cryopreserved at -80°C for later use.

### 2.3 Lentiviral titer determination

Jurkat T cells were inoculated at 1×10⁵/well on a 96-well U-bottom plate, and the collected lentiviral concentrate was diluted in 10-fold increments. 100 µL of virus diluent was added to the corresponding wells, added with an infection promoting agent, i.e., protamine sulfate, to adjust the concentration to 10 µg/mL, centrifuged at 1000×g, 32°C and infected for 90 min, cultured overnight followed by replacement with a fresh RPMI1640 complete medium, and continued to be cultured for 48 h; and the proportion of fluorescence-positive cells was detected by a flow cytometry. The virus titer was calculated using the following formula: Virus titer (TU/mL) = 1×105×proportion of fluorescence-positive cells / 100×1000× corresponding dilution factor.

### Example 3: Preparation of genetically engineered T cells

The following lentiviral vectors obtained by concentration:
LV-EF1α-HER2 CAR-P2A-EGFP;
LV-EF1α-BFP-HATF-HRTE;
LV-EF1α-GFP-RE-mCherry;
LV-EF1α-GFP-HATF-HRTE-RE-mCheny;
LV-EF1α-GFP-HATF-HRTE-RE-CAR; and
LV-EF1α-GFP-HATF-HRTE-RE-BiTE were added to a 48-well flat-bottom plate plated with 1×10⁶ preactivated peripheral blood mononuclear cells at a ratio of MOI=3, added with an infection promoting agent, i.e., protamine sulfate, to adjust the working concentration to 10 µg/mL, centrifuged at 1000×g , 32°C and infected for 90 min, cultured overnight followed by replacement with a fresh T cell growth medium, and continued to be cultured. The fresh T cell growth medium was added every 2-3 days, and the cell density was adjusted to 0.5×10⁶ to 2×10⁶ cells/mL. 6-7 days after infection, immunomagnetic beads of activated T cells were removed, genetically engineered T cells were continued to be cultured and expanded, and subsequent functional experiments could be performed until the cells were resting (9-14 days after removal of the beads).

### Example 4: Preparation of genetically engineered A549 cells

Lentiviral vectors LV-EF1α-GFP-HATF-HRTE-RE-BiTE (MOI = 3) containing 2-6 copies of RE, which were obtained by concentration, were respectively added to a 6-well flat-bottom plate plated with 3×10⁵ A549 cells on alternate days, added with an infection promoting agent, i.e., polybrene, to adjust the working concentration to 10 µg/mL, cultured overnight followed by replacement with a fresh complete medium R10 (RPMI1640 + 10% FBS + 1% PS), and continued to be cultured. After the cells grew to 80% coverage, they were expanded and cultured for later use.

### Example 5: Transcription induction level of the hypoxia-triggered artificial transcription factor of the present invention

In order to verify that the HATF of the present invention had a better ability to induce the expression of exogenous genes than the conventional GAL4-VP64 artificial transcription factor, plasmid transfection experiments were performed for HEK293T cells. The HEK293T cells were plated in a 48-well plate at 5×10⁴ cells/well and transfected the next day after cell attachment. Only 0.25 µg of reaction element plasmid pSV1.0-EGFP-ATR-RE or pSV1.0-EGFP-GAL4UAS which was transfected in a single transfection group; 0.25 µg of pSV1.0-EGFP-RE plasmid and pSV1.0-HATF or pSV1.0-Tat, or 0.25 µg of pSV1.0-EGFP-GAL4UAS plasmid and pSV1.0-GAL4-VP64 which were transfected in an experimental group, were added with a TurboFect transfection reagent in a ratio of plasmids (µg): transfection reagent (µL) of 1: 2, incubated at room temperature for 15-20 min, added to a cell culture plate, and cultured in a 37°C, 5% COz cell incubator for 48 h; and the expression of a gene of interest mCherry was then detected by flow cytometry.

As shown in Fig. 2, the expression positive rate of the gene of interest mCherry in the RE transfection group was only 3.90%, the positive rate of mCherry after HATF induction was as high as 93.3%, and the positive rate after further addition of Tat was as high as 96.9%, while the expression positive rate of the gene of interest mCherry in the GAL4UAS transfection group was 11.4%, and the positive rate of mCherry after GAL4-VP64 induction was 70.9% (Fig. 2a). The positive rate of HATF induction was 22.7 folds that of the reaction element control group (Fig. 2b), and the fluorescence intensity was 110 folds that of the reaction element control group (Fig. 2c). Addition of Tat further improved the induction level, and the folds of induction of the positive rate and the fluorescence intensity were increased to 23.6 and 347, respectively. However, the positive rate of conventional GAL4-VP64 induction was 5.7 times that of the reaction element control group (Fig. 2b), and the fluorescence intensity was only 15 times that of the reaction element control group (Fig. 2c), indicating that the HATF artificial transcription factor constructed in the present invention was superior to the conventional GAL4-VP64 artificial transcription factor in terms of both the background leakage level and the induction ability of the gene of interest.

### Example 6: Expression of fluorescent proteins regulated by hypoxia-sensitive transcription control system

Three different T cells integrating EF1α-GFP-RE-mCherry, EF1α-BFP-HATF-HRTE and EF1α-GFP-RE-mCherry, and EF1α-GFP-HATF-HRTE-RE-mCherry genes were prepared by using the lentiviral expression plasmid in Example 1, the lentiviral vector preparation method in Example 2 and the method in Example 3, which were a RE-mCherry reaction element, a hypoxia-sensitive transcription control system in a binary unit form (co-infected by dual vectors) and a hypoxia-sensitive transcription control system in a unary unit form (injected by a single vector). The above three genetically engineered T cells were cultured under a normoxic condition (21% O₂) and a hypoxic condition (1% O₂) for 24 h; the cells were collected after the culture and eluted with FACS buffer (1× PBS of 2% FBS) once, centrifuged at 500×g for 5 min, then resuspended and mixed uniformly with 300 µL of FACS buffer after the centrifugation; and then, the expression of the red fluorescent protein mCherry was detected by flow cytometer.

The results were shown in Fig. 3. The flow cytometry results of Fig. 3 showed that the background leakage of the hypoxia-sensitive transcription control system in the unary unit form of the present invention was as low as 3.05% in a normoxic environment, and the expression of a high level of red fluorescent protein mCherry was induced in a hypoxic environment, the positive rate was increased to 91.4%, and the mean fluorescence intensity (MFI) was as high as 1480. Interestingly, the hypoxia induction level of the hypoxia-sensitive transcription control system in the binary unit form obtained by co-infection with dual viral vectors was much lower than that of the hypoxia-sensitive transcription control system in the unary unit form obtained by injection with a single virus vector, with the positive rate and fluorescence intensity of mCherry being only 31.8% and 86.6%, which were only 34.8% and 5.85% of the positive rate and mean fluorescence intensity of hypoxia induction in the unary unit form, suggesting that the hypoxia induction effect of the single vector carrying the hypoxia-sensitive transcription control system in the unary unit form was optimal, and the levels of background leakage and hypoxia induction were significantly superior to those of the currently reported dual-vector binary transcription amplification system.

### Example 7: Expression of chimeric antigen receptors regulated by hypoxia-sensitive transcription control system

Two engineered T cells carrying EF1α-HER2 CAR-P2A-EGFP and EF1α-GFP-HATF-HRTE-RE-CAR genes, named HER2 CAR-T cells (positive control) and hypoxia-sensitive HER2 CAR-T cells respectively, were prepared using the lentiviral expression plasmid in Example 1, the lentiviral vector preparation method in Example 2 and the method in Example 3. The above two genetically engineered T cells were cultured under a normoxic condition (21% O₂) or a hypoxic condition (1% O₂) for 24 h respectively; the cells were collected after the culture and eluted with FACS buffer once, added with 2 µg/mL flow cytometry antibody PE-anti-DYKDDDDK, incubated at room temperature in the dark for 20 min, eluted twice with FACS buffer after the incubation, and resuspended and mixed uniformly with 300 µL of FACS buffer; and then, the expression of HER2 CAR molecules was detected by flow cytometer.

The results were shown in Fig. 4. The flow cytometry results showed that HER2 CAR molecules were highly expressed in HER2 CAR-T cells (positive control) under both normoxic and hypoxic conditions, while HER2 CAR molecules were lowly expressed (10.8%) in hypoxia-sensitive HER2 CAR-T cells under the normoxic condition; the expression (62.6%) of the HER2 CAR molecules was up-regulated under the hypoxic condition, and the CAR positive rate and mean fluorescence intensity were increased by a factor of 6 fand 235 respectively, which were far superior to the induction effect of currently reported hypoxia-sensitive CAR-T cells (Juillerat A, Marechal A, Filhol JM, et al. An oxygen sensitive self-decision making engineered CAR T-cell[J]. Sci Rep. 2017, 7:39833*;* Liao Q, He H, Mao Y. et al. Engineering T cells with hypoxia-inducible chimeric antigen receptor (HiCAR) for selective tumor killing. Biomark Res. 2020, 8(1):56*).*

### Example 8: Tumor cell killing of chimeric antigen receptor T cells regulated by hypoxia-sensitive transcription control system

The tumor cell killing efficiency was evaluated by a luciferase-based cytotoxicity assay. First, 1×10⁴ SKOV3-Luc (firefly luciferase gene-modified human ovarian cancer cells) or NCI-H292-Luc (firefly luciferase gene-modified human lung cancer cells) were inoculated on a 96-well flat-bottom black plate with 100 µL of medium per well, and cultured in a 37°C, 5% CO₂ cell incubator for 18 h. On the second day, the genetically engineered T cells in Example 6 and non-transduced T cells cultured at the same time were added to the wells containing target cells at ratios of effector cells to target cells of 1: 2, 1: 1 and 2: 1, and were cultured under a normoxic condition (21% O₂) or a hypoxic condition (1% O₂) for 24 h, respectively; and the luciferase activity value of the target cells was detected by a GloMax^{®}96 microplate luminescence detector after co-culture.

The formula for calculating the cell kill rate was as follows: Cell killing rate (%) = (luciferase activity value of non-transduced T cell group - luciferase activity value of experimental group)/ luciferase activity value of non-transduced T cell group × 100.

The results were shown in Fig. 5. HER2 CAR-T cells (positive control) could effectively kill SKOV3 and NCI-H292 tumor cells under either normoxic or hypoxic condition, and had no selective killing properties. The tumor killing activity of hypoxia-sensitive HER2 CAR-T cells was low under the normoxic condition, and the killing rates were 10.33% (SKOV3) and 13.33% (NCI-H292) at a ratio of effector cells to target cells of 2: 1, while SKOV3 tumor cells (67.67%) and NCI-H292 tumor cells (93.33%) could be selectively and efficiently killed under the hypoxic environment; and the killing activity against tumor cells was increased by a factor of 6.

### Example 9: In vivo safety of hypoxia-sensitive CAR-T cells

A recombinant adenovirus (HER2-Ad) was diluted with 1 × PBS to a target dose of 8×10⁹ TU/mL;
125 µL of HER2-Ad was intraperitoneally injected with a 1 mL syringe, and 7 days later, the expression of luciferase was detected by a small animal in vivo imager; positive mice were randomly selected and grouped, and except for 13 mice in the HER2 CAR-T cell infusion group, each of the remaining groups has 4 mice for subsequent experiments; and
5×10⁶ HER2 CAR-T cells, hypoxia-sensitive HER2 CAR-T cells and control cells were intravenously infused. Every 2-3 days, the mice were weighed, the orbital vein blood was collected to detect serum cytokines, and the health status of the mice was observed.

The results were shown in Fig. 6. Control HER2 CAR-T cells caused a progressive decrease in body weight (Fig. 6a), increased mortality (Fig. 6b) and impaired liver function of the mice caused a large amount of alanine aminotransferase (ALT) into the blood (Fig. 6c), and released a large number of cytokines related to T cell activation into the blood (Fig. 6d), while hypoxia-sensitive HER2 CAR-T cells did not cause the above toxic and side effects, suggesting that they did not cause on-target offtumor toxicity.

### Example 10: In vivo antitumor effects of hypoxia-sensitive CAR-T cells

Local hair removal on the back of B-NDG mice raised in a sterile isolator was performed with a hair removal cream or an animal shaver one day in advance, such that the skin of a tumor cell inoculation site was exposed.

Each mouse was fixed with the left hand, that is, the head, neck and back skin of the mouse were grasped with the left hand at the same time; and after a part to be hair-removed on the right side of the back was fully exposed by turning its back to the left, this part was disinfected by an alcohol cotton ball with the right hand. A 1 mL insulin syringe was used to blow and mix uniformly the pre-prepared SKOV3 or NCI-H292 tumor cells, and then suck 125 µL of cell suspension (5×10⁶ tumor cells), and a tip of a needle was pierced subcutaneously and diagonally into the mouse at an angle of 30°-40° from the skin; and the cell suspension was slowly injected to avoid cell spillage. After the injection of 125 µL of cell suspension was completed, the needle was left for 2-3 seconds and then quickly withdrawn, and a clearly visible bulge could be seen under the skin at the injection site.

After cell inoculation, the tumorigenesis and health status of the mice were observed every 2-3 days, and the baseline tumor volume was measured with a vernier caliper after tumorigenesis, and subsequent experiments were carried out.

5×10⁶ hypoxia-sensitive HER2 CAR-T cells and control cells were intravenously infused, the tumor size was measured every 2-3 days, and a long diameter and a short diameter of the tumor were measured with a vernier caliper, respectively; and the tumor volume was calculated according to the following formula: volume = (long diameter × short diameter²)/2.

The results were shown in Fig. 7. HER2 CAR-T cells (hypoxia-sensitive HER2 CAR-T cells) controlled by the hypoxia-sensitive transcription control system could effectively inhibit the growth of SKOV3 (ovarian cancer) and NCI-H292 (lung cancer), with tumor suppression rates of up to 100% and 78.5% on day 35 after cell infusion.

### Example 11: Hypoxia-triggered expression of CD47/CD3 bispecific T cell engager protein

Genetically engineered A549 cells in Example 4 were plated in a 12-well flat-bottom plate one day in advance, with 3×10⁵ cells per well and a total volume of 1 mL.

On the second day, the plate was cultured under a normoxic condition (21% O₂) or a hypoxic condition (1% O₂) for 24 h; the cells were collected after culture, resuspended with 1×BS, diluted into 1× with 4×SDS Loading Buffer, and fully blown and mixed uniformly; and the sample was placed in a pot, and heated and boiled with an induction cooker for 10 min to fully denature the protein.

According to the molecular weight (72.5 kD) of the hypoxia-sensitive artificial transcription factor and the molecular weight (54.7 kD) of BiTE of a protein of interest, upper and lower layers of SDS-PAGE gel with a concentration of 10% were prepared, and the sample was separated by electrophoresis in the SDS-PAGE gel for 2 h; and then the sample was transferred from the gel to a PVDF membrane, which is activated by methanol in advance, by means of wet transfer.

According to the instructions of a protein Marker, the PVDF membrane containing the protein of interest and a reference protein was cut and placed in a 5% skimmed milk powder blocking solution, and incubated on a shaker for at least 1 h at room temperature.

The blocked PVDF membrane was taken out and placed in a mouse anti-His-tag primary antibody diluent, with a dilution ratio of primary antibody of 1: 1000, and placed on a shaker at 4°C overnight.

The primary antibody diluent was recovered and cryopreserved at -20°C for later use. The PVDF membrane was taken out, and oscillated and eluted with PBST on the shaker for three times for 8 min each time, and a corresponding goat anti-mouse IgG-HRP secondary antibody diluent, with a dilution ratio of secondary antibody of 1: 3000, was added after elution was completed, and incubated on the shaker at room temperature for 1 h.

After the PVDF membrane was taken out, it was eluted with PBST for three times, and then exposed after color development.

The results were shown in Fig. 8. Fig. 8 showed the exposure result of western blotting detection, wherein the hypoxia-sensitive artificial transcription factor and a downstream gene of interest CD47/CD3-BiTE were almost not expressed under a normoxic condition, but under a hypoxic environment, the expression of the hypoxia-sensitive artificial transcription factor could be induced and the expression of the downstream gene of interest CD47/CD3-BiTE could be initiated. In addition, tandem repeats of 2-6 copies of the core sequence did not affect the regulatory activity of the hypoxia-sensitive transcription control system, and could achieve degradation in the normoxic environment, and efficiently-induced expression of the protein of interest in the hypoxic environment.

### Example 12: Tumor cell killing of CD47/CD3-BiTE-T cells regulated by hypoxia-sensitive transcription control system

The tumor cell killing efficiency detection method and the killing efficiency calculation were the same as those in Example 7. First, 1×10⁴ SKOV3-Luc (firefly luciferase gene-modified human ovarian cancer cells) or NCI-H292-Luc (firefly luciferase gene-modified human lung cancer cells) were inoculated on a 96-well flat-bottom black plate with 100 µL of medium per well, and cultured in a 37°C, 5% CO₂ cell incubator for 18 h. On the second day, the hypoxia-sensitive CD47/CD3-BiTE-T cells in Example 8 and non-transduced T cells cultured at the same time were added to the wells containing target cells at ratios of effector cells to target cells of 1: 2, 1:1, and 2: 1, and were cultured under a normoxic condition (21% O₂) or a hypoxic condition (1% O₂) for 20 h, respectively; and the luciferase activity value of the target cells was detected by a GloMax^{®}96 microplate luminescence detector after co-culture.

The results were shown in FIG. 9. The tumor killing activity of hypoxia-sensitive CD47/CD3-BiTE-T cells was low under the normoxic condition, and the killing rates were 2.67% (SKOV3) and 3.33% (NCI-H292) at a ratio of effector cells to target cells of 2: 1, while SKOV3 tumor cells (78.67%) and NCI-H292 tumor cells (97.67%) could be selectively and efficiently killed under the hypoxic condition; and the killing activity was increased by a factor of approximately 30.

### Example 13: In vivo antitumor effect of hypoxia-sensitive CD47/CD3-BiTE-T cells

First, 5×10⁶ NCI-H292-Luc (human lung cancer cells modified by firefly luciferase gene) were inoculated subcutaneously on the right side of the back of B-NDG mice, and each injected with 125 µL of tumor cell suspension; hypoxia-sensitive BiTE-T cells prepared in Example 3 and non-transduced T cells cultured at the same time were injected intravenously or intratumorally day 6 after tumor inoculation; and 5×10⁶ positive cells were infused into each tumor-bearing mouse. After the tumor cells were inoculated, a long diameter and a short diameter of the tumor were measured by a vernier caliper, and the tumor volume was calculated according to the following formula: tumor volume = (long diameter × short diameter²)/2.

The results were shown in Fig. 10: both intravenous infusion and intratumoral injection of hypoxia-sensitive CD47/CD3-BiTE-T cells could effectively control the growth of lung cancer; and tumor suppression rates one month after cell infusion were 52.35% (intravenous infusion) and 78.69% (intratumoral injection), respectively, compared with the non-transduced T cell treatment group.

The above examples are exemplary, and should not be construed as limiting the present invention. A person of ordinary skill in the art can make changes, modifications, substitutions and variations to the above examples within the scope of the present invention.

## Claims

1. A hypoxia-triggered artificial transcription factor HATF, the HATF comprising:
(1) a sequence shown in SEQ ID NO: 1;
(2) a sequence having one or more amino acid substitutions, deletions or additions compared with the sequence shown in SEQ ID NO: 1; or
(3) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in SEQ ID NO: 1.

2. A nucleic acid sequence encoding the HATF according to claim 1.

3. A recognition element (RE) for the HATF according to claim 1, the RE comprising a core sequence selected from:
(1) a sequence shown in SEQ ID NO: 2, or its complementary sequence;
(2) a sequence hybridized to the sequence shown in SEQ ID NO: 2 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 2; or
(4) a sequence obtained by derivatization of the sequence shown in SEQ ID NO: 2 by deletion, substitution, insertion, or addition of one or more nucleotides;
preferably, the RE comprises a plurality of copies of the core sequence and a minimal promoter; more preferably, the plurality of copies are two copies, three copies, four copies, five copies, six copies, seven copies, eight copies, nine copies or ten copies; further preferably, the recognition element (RE) comprises five or six copies of the core sequence and a minimal promoter;
still further preferably, the RE comprises a sequence selected from:
(1) a sequence shown in SEQ ID NO: 3, or its complementary sequence;
(2) a sequence hybridized to the sequence shown in SEQ ID NO: 3 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 3; or
(4) a sequence obtained by derivatization of the sequence shown in SEQ ID NO: 3 by deletion, substitution, insertion, or addition of one or more nucleotides; and
most preferably, the RE is shown in SEQ ID NO: 3.

4. A hypoxia-triggered transcription control system, wherein the transcription control system comprises the nucleic acid sequence according to claim 2 and the RE according to claim 3; and
preferably, the hypoxia-triggered transcription control system comprises two sets of transcription control units linked upstream and downstream, wherein the upstream transcription control unit comprises a hypoxia-triggered transcription reaction element HRTE for controlling the HATF and a nucleic acid sequence encoding the HATF, and the downstream transcription control unit comprises the RE and a gene of interest GOI.

5. The hypoxia-triggered transcription control system according to claim 4, wherein the transcription control units linked upstream and downstream are nonlinearly connected in series, and located at two vectors;
preferably, the transcription control system has a combined form shown in any one of the following formulas:
HATF-HRTE and RE-GOI;
HATF-HRTE and GOI-RE;
HRTE-HATF and GOI-RE; or
HRTE-HATF and RE-GOI;
or the transcription control units linked upstream and downstream are linearly connected in series, and located at the same vector; and
preferably, the transcription control system has a combined form shown in any one of the following formulas:
HATF-HRTE-RE-GOI;
HATF-HRTE-GOI-RE;
HRTE-HRE-GOI;
HRTE-HATF-GOI-RE;
RE-GOI-HATF-HRTE;
RE-GOI-HRTE-HATF;
GOI-RE-HATF-HRTE; or
GOI-RE-HRTE-HATF.

6. The hypoxia-triggered transcription control system according to claim 4 or 5, wherein the HRTE is selected from a partial sequence of a hypoxia-inducible gene; preferably, the HRTE is selected from a flanking region of a vascular endothelial growth factor gene, an erythropoietin gene and a glycolytic enzyme gene; more preferably, a core sequence of the HRTE is 5'-(A/G)CGT(G/C)-3';
preferably, the HRTE comprises a core sequence selected from:
(1) a sequence shown in SEQ ID NO: 4, or its complementary sequence;
(2) a sequence hybridized to the sequence shown in SEQ ID NO: 4 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 4; or
(4) a sequence obtained by derivatization of the sequence shown in SEQ ID NO: 4 by deletion, substitution, insertion, or addition of one or more nucleotides;
preferably, the HRTE comprises a plurality of copies of the core sequence and a minimal promoter; more preferably, the plurality of copies are two copies, three copies, four copies, five copies, six copies, seven copies, eight copies, nine copies or ten copies; further preferably, the HRTE comprises five or six copies of the core sequence and a minimal promoter;
still further preferably, the HRTE comprises a sequence selected from:
(1) a sequence shown in SEQ ID NO: 5, or its complementary sequence;
(2) a sequence hybridized to the sequence shown in SEQ ID NO: 5 under strict hybridization conditions;
(3) a sequence having a sequence identity of at least 85%, 90%, 91%, 92%, 93%, 94%, 95%, 96%, 97%, 98%, 98.5%, 99%, or 99.5% with the sequence shown in SEQ ID NO: 5; or
(4) a sequence obtained by derivatization of the sequence shown in SEQ ID NO: 5 by deletion, substitution, insertion, or addition of one or more nucleotides; and
most preferably, the HRTE is shown in SEQ ID NO: 5.

7. The hypoxia-triggered transcription control system according to any one of claims 4 to 6, wherein the GOI is one or more selected from:
a cytokine and a chemokine, preferably selected from GM-CSF, IFN-α/β/γ, IL-2, IL-3, IL-7, IL-12, IL-15, IL-21, IL-33, IL-35, IL-37, CCL4, CCL20, CXCL9, CXCL10, CXCL11, CXCL12, CXCL13, MIP-1α, or MIP-1β;
a cytotoxic molecule, preferably selected from TNF-α, T cell engagers against different antigen targets, chimeric antigen receptors against different antigen targets, an apoptotic gene, a pyroptosis gene, or a toxin;
agonistic antibodies against different antigen targets, preferably selected from an anti-CD28 antibody, an anti-4-1BB antibody, an anti-ICOS antibody, an anti-GITR antibody, an anti-OX40 antibody or an anti-CD27 antibody; or
blocking antibodies against different antigen targets, preferably selected from a CTLA-4 antibody, a PD-1 antibody, a PD-L1 antibody, a LAG-3 antibody or a Tim3 antibody; or
a combination of any two or more of the above;
more preferably, the corresponding antigen targets are one or more selected from AXL, EGFR, MHC, CD24, CD47, FAP, CD147, HER-2, CD55, CD59, ROR1, ROR2, CD133, CD44v6, CD44v7, CD44v8, CD126, CD171, CEA, EpCAM, TAG72, IL-13Rα, EGFRvIII, GD2, GD3, FRα, PSCA, PSMA, GPC3, CAIX, Claudin18.2, VEGFR2, PD-L1, PD-L2, MSLN, MUC1, c-Met, FOLR1, B7-H3 and Trop2; and
preferably, an expression cassette of the GOI comprises an amino acid sequence shown in any one of SEQ ID NOs: 6-8.

8. A nucleic acid sequence comprising the hypoxia-triggered transcription control system according to any one of claims 4 to 7, wherein
preferably, the nucleic acid sequence is shown in SEQ ID NO: 9.

9. A vector comprising the nucleic acid sequence according to claim 8, wherein
preferably, the vector is selected from a plasmid, a retroviral vector, a lentiviral vector, an adenoviral vector, an adeno-associated viral vector, a vaccinia virus vector, a herpes simplex virus vector, a forest encephalitis virus vector, a poliovirus vector, a Newcastle disease virus vector, a transposon or a combination of one or more thereof; and more preferably, the vector is a lentiviral vector.

10. A host cell, the host cell comprising the vector according to claim 9 or the nucleic acid molecule according to claim 8 being integrated into a chromosome of the host cell, wherein
preferably, the host cell is an isolated human-derived cell, more preferably selected from an embryonic stem cell, an umbilical cord blood-derived stem cell, an induced pluripotent stem cell, a hematopoietic stem cell, a mesenchymal stem cell, an adipose-derived stem cell, a T cell, an NK cell, an NKT cell or a macrophage; further preferably, the human-derived cell is a genetically engineered immune cell; still further preferably, the genetically engineered immune cell is a T cell, an NK cell, an NKT cell or a macrophage; still further preferably, the genetically engineered immune cell is a T cell; and
most preferably, the genetically engineered immune cell is selected from the group consisting of:
a chimeric antigen receptor T cell (CAR-T cell);
a chimeric antigen receptor NK cell (CAR-NK cell);
a chimeric antigen receptor NKT cell (CAR-NKT cell);
a chimeric antigen receptor macrophage (CAR- mø); and
a T cell receptor T cell (TCR-T cell).

11. Use of the hypoxia-triggered artificial transcription factor HATF according to claim 1, the recognition element (RE) according to claim 3, the hypoxia-triggered transcription control system according to any one of claims 4 to 7, the nucleic acid molecule according to claim 8, the vector according to claim 9, or the host cell according to claim 10 in the preparation of a medicament or a formulation for treating hypoxic diseases, ischemic diseases, or cancers, wherein
preferably, the cancer is a solid tumor; and
more preferably, the solid tumor is one or more selected from neuroblastoma, lung cancer, breast cancer, esophageal cancer, gastric cancer, liver cancer, cervical cancer, ovarian cancer, kidney cancer, pancreatic cancer, nasopharyngeal cancer, small bowel cancer, large bowel cancer, colorectal cancer, bladder cancer, bone cancer, prostate cancer, thyroid cancer or brain cancer.

12. A method for treating hypoxic diseases, ischemic diseases or cancers, the method comprising: administrating to a subject in need thereof a therapeutically effective amount of the hypoxia-triggered artificial transcription factor HATF according to claim 1, the recognition element (RE) according to claim 3, the hypoxia-triggered transcription control system according to any one of claims 4 to 7, the nucleic acid molecule according to claim 8, the vector according to claim 9, or the host cell according to claim 10.
